# EUROPÄISCHE PATENTANMELDUNG

(11) **EP 0 821 236 A2**
(43) Veröffentlichungstag der Anmeldung: **28.01.1998**
(21) Anmeldenummer: 97112363.3
(22) Anmeldetag: 18.07.1997
(51) Int. Cl.: G01N 33/52

(54) **Diagnostischer Testträger und Verfahren zur Bestimmung eines Analyts mit dessen Hilfe**

(30) Priorität: 23.07.1996 DE 19629655
(71) Anmelder: BOEHRINGER MANNHEIM GMBH, 68298 Mannheim (DE)
(72) Erfinder: Vetter, Peter, 67105 Schifferstadt (DE); Leininger, Helmut, 68259 Mannheim (DE)

(57) **Zusammenfassung**

Gegenstand der Erfindung ist ein diagnostischer Testträger (1) enthaltend eine Tragschicht (2) mit einer darauf angeordneten zur Bestimmung eines Analyts in flüssiger Probe erforderliche Reagenzien enthaltende Nachweisschicht (3) und ein die Nachweisschicht überdeckendes Netzwerk (4), das größer als die Nachweisschicht (3) ist und das auf der Tragschicht (2) befestigt ist, dadurch gekennzeichnet, daß das Netzwerk (4) ein mehrfädiges Gewirke ist, dessen Fäden an der der Nachweisschicht (3) zugewandten Seite des Gewirkes stark aufgerauht und die Fäden an der die Nachweisschicht (3) abgewandten Seite des Gewirkes relativ glatt sind sowie die Verwendung eines solchen Testträgers zur Bestimmung eines Analyts in einer Flüssigkeit
und die Verwendung eines entsprechenden Gewirkes als Flüssigkeit verteilende Schicht in einem diagnostischen Testträger. Außerdem ist Gegenstand der Erfindung ein Verfahren zur Bestimmung eines Analyts in flüssiger Probe mit Hilfe eines erfindungsgemäßen Testträgers.

## Beschreibung

Die Erfindung betrifft einen diagnostischen Testträger enthaltend eine Tragschicht mit einer darauf angeordneten zur Bestimmung eines Analyts in flüssiger Probe erforderliche Reagenzien enthaltende Nachweisschicht und ein die Nachweisschicht überdeckendes Netzwerk, das größer als die Nachweisschicht ist und das auf der Tragschicht befestigt ist. Die Erfindung betrifft außerdem die Verwendung dieses diagnostischen Testträgers zur Bestimmung eines Analyts in einer Flüssigkeit und die Verwendung eines Flüssigkeit aufnehmen Gewirkes als Flüssigkeit verteilende Schichten in einem diagnostischen Testträger.

Zur qualitativen oder quantitativen analytischen Bestimmung von Bestandteilen von Körperflüssigkeiten, insbesondere von Blut, werden oft sogenannte trägergebundene Tests verwendet. Bei diesen sind Reagenzien in entsprechenden Schichten eines festen Testträgers eingebettet, der mit der Probe in Kontakt gebracht wird. Die Reaktion von flüssiger Probe und Reagenzien führt zu einem nachweisbaren Signal, insbesondere einer Farbänderung, welche visuell oder mit Hilfe eines Geräts, meistens reflektionsphotometrisch, ausgewertet werden kann.

Testträger sind häufig als Teststreifen ausgebildet, die im wesentlichen aus einer länglichen Tragschicht aus Kunststoffmaterial und darauf angebrachten Nachweisschichten als Testfelder bestehen. Es sind jedoch auch Testträger bekannt, die als quadratische oder rechteckige Plättchen gestaltet sind.

Testträger der eingangs bezeichneten Art sind beispielsweise aus der deutschen Patentschrift 21 18 455 bekannt. Dort werden diagnostische Testträger zum Nachweis von Analyten in Flüssigkeiten beschrieben, die aus einer Tragschicht und mindestens einer die Nachweisreagenzien enthaltenden Nachweisschicht, deren nicht auf der Tragschicht anliegende Oberfläche mit einer Deckschicht versehen ist, bestehen. Die Deckschicht kann aus einem feinmaschigen Netzwerk in Form eines Gewebes, Gewirkes oder Vlieses bestehen. Kunststoffgewebe werden als bevorzugte Netzwerke angegeben, um eine schnelle Benetzung der Nachweisschicht mit Probenflüssigkeit zu erreichen und störende Chromatographieeffekte zu vermeiden. Zum Nachweis eines Analyts in einer Flüssigkeit wird ein solcher diagnostischer Testträger in eine entsprechende Flüssigkeit eingetaucht. Die Nachweisschicht kommt so mit einem sehr großen Überschuß an Flüssigkeit in Kontakt, der nicht von dem Testträger aufgenommen werden kann. Je nach der Dauer des Kontaktes der Nachweisschicht mit der zu untersuchenden Flüssigkeit können jedoch unterschiedliche Farbintensitäten beobachtet werden. Längere Kontaktzeiten führen in der Regel zu um so positiveren Resultaten. Eine korrekte quantitative Analytbestimmung ist deshalb so nicht möglich.

Aufgabe der vorliegenden Erfindung ist es, einen diagnostischen Testträger zur quantitativen Bestimmung eines Analyts in einer Flüssigkeit zur Verfügung zu stellen, auf den eine undosierte Probenflüssigkeitsmenge aufgegeben werden kann und bei dem ein Probenflüssigkeitsüberschuß nicht zu zeitabhängigen falsch-positiven Resultaten führt.

Diese Aufgabe wird durch die in den Patentansprüchen näher charakterisierte Erfindung gelöst.

Gegenstand der Erfindung ist nämlich ein diagnostischer Testträger mit einer Tragschicht und darauf angeordneten Nachweisschicht, welche zur Bestimmung eines Analyts in flüssiger Probe erforderliche Reagenzien enthält. Die Nachweisschicht überdeckt ein Netzwerk, das größer als die Nachweisschicht ist und das außerhalb der Nachweisschicht auf der Tragschicht befestigt ist. Das Netzwerk des erfindungsgemäßen diagnostischen Testträgers ist ein mehrfädiges Gewirke, dessen Fäden an der der Nachweisschicht zugewandten Seite stark aufgerauht sind, während die Fäden an der der Nachweisschicht abgewandten Seite des Gewirkes glatt sind.

Gegenstand der Erfindung ist außerdem die Verwendung eines solchen diagnostischen Testträgers zur Bestimmung eines Analyts in einer Flüssigkeit. In sofern ist auch ein Verfahren zur Bestimmung eines Analyts in flüssiger Probe mit Hilfe eines solchen diagnostischen Testträgers Gegenstand der Erfindung, bei dem soviel Probenflüssigkeit auf die der Nachweisschicht abgewandten Seite des Netzwerkes aufgegeben wird, daß die Nachweisschicht mit Flüssigkeit gesättigt wird. Das Netzwerk führt dann überschüssige Flüssigkeit von der Nachweisschicht in den die Nachweisschicht überragenden Bereich des Netzwerkes, woraufhin dann die Nachweisschicht auf eine Farbveränderung hin beobachtet wird. Die Intensität der Farbveränderung ist ein Maß für die Menge an Analyt in der untersuchten flüssigen Probe.

Schließlich ist auch Gegenstand der Erfindung die Verwendung eines Flüssigkeit aufnehmenden, flächig ausgebildeten Gewirkes mit einem maschenbildenden Faden aus einem vollsynthetischen Material und aus als Schußfäden in das Gewirke eingearbeiteten hydrophilen Fäden aus saugfähigen Garnen, die auf einer Seite des Gewirkes aufgerauht sind als Flüssigkeit verteilende Schicht in einem diagnostischen Testträger. Ein solches Gewirke ist beispielsweise aus der deutschen Patentschrift 32 13 673 aus einem völlig anderen technischen Gebiet, nämlich als wundseitig nicht haftendes Wundtextil mit Wundsekret aufnehmenden Eigenschaften bekannt. Die dort genannten Merkmale des Gewirkes treffen auch für das erfindungsgemäß einsetzbare Netzwerk zu. So ist dieses Netzwerk ein elastisches Gewirke mit einem maschenbildenden Faden aus einem ersten Material und mit als Schußfäden eingearbeiteten Fäden aus einem sehr saugfähigen zweiten Material. Das zweite Material ist vorteilhafterweise hydrophiler als das erste Material. Dies kann dadurch erreicht werden, daß das erste Material vor allem aus vollsynthetischem Material wie beispielsweise Polyester, Polyamid oder Polypropylen besteht. Das zweite Material dagegen sollte aus hochsaugfähigen Garnen wie beispielsweise aus Baumwolle, Zellwolle oder Leinen bestehen. Das erfindungsgemäß einsetzbare Gewirke zeichnet sich unter anderem auch dadurch aus, daß sich der maschenbildende Faden auf der der Nachweisschicht abgewandten Seite des Gewirkes befindet und die Schußfäden auf der der Nachweisschicht zugewandten Seite des Gewirkes.

Überraschenderweise eignet sich das aus dem Stand der Technik lediglich als Wundtextil bekannte Gewirke in einem diagnostischen Testträger hervorragend dafür, Flüssigkeit schnell und gleichmäßig zu verteilen und überschüssige Flüssigkeit von einer darunter liegenden Schicht wegzuführen, wenn das Netzwerk größer als diese darunter liegende Schicht ist, so daß die überschüssige Flüssigkeit in den die darunter liegende Schicht überragenden Teil des Netzwerkes aufgenommen werden kann.

In einem erfindungsgemäßen diagnostischen Testträger kommen für die Tragschicht insbesondere solche Materialien in Frage, die die zu untersuchenden Flüssigkeiten nicht aufnehmen. Dies sind sogenannte nicht-saugfähige Materialien, wobei Kunststoffolien beispielsweise aus Polystyrol, Polyvinylchlorid, Polyester oder Polyamid besonders bevorzugt sind. Es ist jedoch auch möglich, saugfähige Materialien, wie zum Beispiel Holz, Papier oder Pappe mit wasserabstoßenden Mitteln zu imprägnieren oder mit einem wasserfesten Film zu überziehen, wobei als Hydrophobierungsmittel Silikone oder Hartfette und als Filmbildner beispielsweise Nitrocellulose oder Celluloseacetat verwendet werden können. Als weitere Trägermaterialien eignen sich Metallfolien oder Glas.

Für die Nachweisschicht ist es im Gegensatz hierzu erforderlich, solche Materialien einzusetzen, die in der Lage sind, die zu untersuchende Flüssigkeit mit darin enthaltenen Inhaltsstoffen aufzunehmen. Dies sind sogenannte saugfähige Materialien, wie beispielsweise Vliese, Gewebe, Gewirke oder poröse Kunststoffmaterialien, die als Schichtmaterialien verwendet werden können. Die für die Nachweisschicht in Frage kommenden Materialien müssen natürlich auch Reagenzien tragen können, die für den Nachweis des zu bestimmenden Analyts erforderlich sind. Im einfachsten Fall befinden sich alle für den Nachweis des Analyts erforderliche Reagenzien auf einer Schicht. Es sind jedoch auch Fälle vorstellbar, für die es vorteilhafter ist, die Reagenzien auf mehrere saugfähige Materialschichten zu verteilen, die dann übereinander, sich vollflächig berührend angeordnet sind. Der im folgenden verwendete Begriff "Nachweisschicht" soll sowohl solche Fälle umfassen, bei denen sich die Reagenzien entweder nur in oder auf einer Schicht oder in zwei oder noch mehr, wie vorstehend beschrieben, angeordneten Schichten befinden.

Außerdem kann die Nachweisschicht auch eine Schicht enthalten, die Plasma oder Serum aus Vollblut abzutrennen in der Lage ist, wie beispielsweise ein Glasfaservlies, wie es zum Beispiel aus EP-B-0 045 476 bekannt ist. Eine oder mehrere solcher Abtrennschichten kann über einer oder mehrere Schichten, die Nachweisreagenzien tragen, liegen. Auch ein solcher Aufbau soll von dem Begriff "Nachweisschicht" umfaßt sein.

Bevorzugte Materialien für die Nachweisschicht sind Papiere oder poröse Kunststoffmaterialien, wie Membranen. Besonders bevorzugt sind asymmetrisch poröse Membranen, die vorteilhafterweise so angeordnet sind daß die zu untersuchende Probenflüssigkeit auf die großporige Seite der Membran aufgegeben wird und die Bestimmung des Analyts von der feinporigen Seite der Membran aus erfolgt. Als poröse Membranmaterialien sind Polyamid-, Polyvinylidendifluorid-, Polyethersulfon- oder Polysulfonmembranen ganz besonders bevorzugt. Für den erfindungsgemäßen Testträger sind insbesondere Polyamid 66-Membranen hervorragend geeignet. Die Reagenzien zur Bestimmung des nachzuweisenden Analyts sind in der Regel durch Imprägnierung in die vorstehend genannten Materialien eingebracht worden.

Für die Nachweisschicht kommen jedoch auch sogenannte offene Filme in Frage, wie sie beispielsweise in EP-B-0 016 387 beschrieben sind. Hierfür werden einer wäßrigen Dispersion von flimbildenden organischen Kunststoffen Feststoffe als feine, unlösliche, organische oder anorganische Partikel zugegeben und die für die Nachweisreaktion erforderlichen Reagenzien zusätzlich hinzugefügt. Geeignete Filmbildner sind bevorzugt organische Kunststoffe, wie Polyvinylester, Polyvinylacetate, Polyacrylester, Polymethacrylsäure, Polyacrylamide, Polyamide, Polystyrol, Mischpolymerisate, zum Beispiel von Butadien und Styrol oder von Maleinsäureester und Vinylacetat oder andere filmbildende, natürliche und synthetische organische Polymere sowie Mischungen derselben in Form von wäßrigen Dispersionen. Die Dispersionen lassen sich auf einer Unterlage zu einer gleichmäßigen Schicht verstreichen, die nach dem Trocknen einen wasserfesten Film ergibt. Die trocknen Filme haben eine Dicke von 10 µm bis 500 µm, vorzugsweise von 30 bis 200 µm. Der Film kann mit der Unterlage als Träger zusammen verwendet werden oder für die Nachweisreaktion aufeinen anderen Träger aufgebracht werden. Obwohl die für die Nachweisreaktion erforderlichen Reagenzien normalerweise in die zur Herstellung der offenen Filme verwendete Dispersion gegeben werden, kann es auch vorteilhaft sein, wenn der gebildete Film nach seiner Herstellung mit den Reagenzien imprägniert wird. Auch eine Vorimprägnierung der Füllstoffe mit den Reagenzien ist möglich. Welche Reagenzien zur Bestimmung eines bestimmten Analyts eingesetzt werden können sind dem Fachmann bekannt. Dies muß hier nicht näher ausgeführt werden.

In dem erfindungsgemäßen diagnostischen Testträger ist das die Nachweisschicht überdeckende Netzwerk größer als die darunter liegende Nachweisschicht. Der die Nachweisschicht überragende Teil des Netzwerkes ist auf der Tragschicht befestigt. Die Befestigung kann nach dem Fachmann aus der Testträgertechnologie bekannten Methoden erfolgen. Beispielsweise kann die Befestigung mittels Heißsiegelschichten (Polyethylen), Schmelzkleber oder härtendem Kaltkleber erfolgen. Als vorteilhaft haben sich auch doppelseitig klebende Streifen erwiesen. In allen Fällen ist es jedoch wichtig, daß die Befestigung des Netzwerkes auf der Tragschicht so erfolgt, daß von der Nachweisschicht ein kapillaraktiver Flüssigkeitstransport in den Teil des Netzwerkes möglich ist, der auf der Tragschicht befestigt ist. Dieser kapillaraktive Flüssigkeitstransport muß insbesondere dann möglich sein, wenn die Nachweisschicht mit Flüssigkeit gesättigt ist.

Zur Bestimmung des in der Probenflüssigkeit nachzuweisenden Analyts ist in dem erfindungsgemäßen diagnostischen Testträger die Nachweisschicht durch die Tragschicht sichtbar. Dies kann dadurch erreicht werden, daß die Tragschicht transparent ist. Es ist aber auch möglich, daß die Tragschicht ein Loch aufweist, das von der Nachweisschicht überdeckt ist. Durch das Loch ist dann die Nachweisschicht sichtbar. In einer bevorzugten Ausführungsform des erfindungsgemäßen diagnostischen Testträgers befindet sich in der Tragschicht unterhalb der Nachweisschicht ein Loch, durch das die Nachweisschicht beobachtbar ist. Das Loch besitzt einen etwas kleineren Durchmesser als die kleinste Längenausdehnung der Nachweisschicht, so daß die Nachweisschicht außerhalb des Loches auf der Tragschicht aufliegt und dort befestigt sein kann. In der Regel ist die Nachweisschicht durch ein doppelseitiges Klebeband und das darüber liegende Netzwerk und dessen Befestigung auf der Tragschicht ausreichend fixiert.

Zur Durchführung eines Verfahrens zur Bestimmung eines Analyts in flüssiger Probe mit Hilfe eines erfindungsgemäßen diagnostischen Testträgers wird soviel Probenflüssigkeit auf die der Nachweisschicht abgewandte Seite des Netzwerkes aufgegeben, daß die durch das Netzwerk gelangende Flüssigkeit die Nachweisschicht vollständig sättigt. Als Probenflüssigkeit kommen insbesondere Körperflüssigkeiten wie Blut, Plasma, Serum, Urin, Speichel etc. in Frage. Blut oder von Blut abgeleitete Flüssigkeiten wie Plasma oder Serum sowie Urin sind besonders bevorzugte Probenflüssigkeiten. Überschüssige Flüssigkeit wird durch das Netzwerk von der Nachweisschicht in den die Nachweisschicht überragenden Bereich des Netzwerkes weggeführt. In der Nachweisschicht kann dann bei Anwesenheit des zu bestimmenden Analyts ein Signal nachgewiesen werden. Vorteilhafterweise handelt es sich bei einem solchen Signal um eine Farbänderung, worunter sowohl Farbbildung, Farbverlust als auch Farbumschlag verstanden wird. Die Intensität der Farbänderung ist ein Maß für die Menge an Analyt in der untersuchten flüssigen Probe. Sie kann visuell oder mit Hilfe eines Gerätes, meistens reflektionsphotometrisch quantitativ ausgewertet werden.

Zur Markierung der Probenflüssigkeitsauftragsstelle kann vorteilhafterweise eine Abdeckung der über die Nachweisschicht hinausragenden Bereiche des Netzwerkes erfolgen. Hierfür können beispielsweise zwei wasserundurchlässige inerte Schichtmaterialien so auf dem Netzwerk befestigt werden, daß der Bereich des Netzwerkes, der über dem Loch unter der Nachweisschicht in der Tragschicht liegt, zur Probenaufgabe frei bleibt. Als besonders günstig hat es sich erwiesen, wenn diese Schichtmaterialien Klebefolien sind, die nicht nur die über die Nachweisschicht hinausragenden Bereiche des Netzwerkes mit ihrer Klebeseite kontaktieren, sonder darüberhinaus auch noch auf der Tragschicht befestigt sind. Neben der Markierung der Probenflüssigkeitsauftragsstelle unterstützt eine solche Abdeckung auch noch die Kapillarkräfte, die ein Wegleiten überschüssiger Flüssigkeit von der Nachweisschicht bewirken. Außerdem führt die Abdeckung dazu, daß von der Nachweisschicht weggeleitete überschüssige Flüssigkeit von Kontakt geschützt ist und solche Flüssigkeit nicht leicht von dem Testträger herabtropfen kann.

Ein großer Vorteil des erfindungsgemäßen diagnostischen Testträgers besteht darin, daß kein vorbestimmtes Volumen einer Probenflüssigkeit auf den Testträger aufgegeben werden muß. Es ist lediglich sicherzustellen, daß ein Überschuß verwendet wird, so daß die Nachweisschicht vollständig mit Flüssigkeit gesättigt wird. Überschüssige Flüssigkeit wird, wie bereits vorstehend erwähnt, durch das über die Nachweisschicht hinausragenden Netzwerk von der Nachweisschicht weggeleitet. Weil überschüssige Flüssigkeit von der Nachweisschicht weggeleitet wird, wird auch hygienischen Gesichtspunkten Rechnung getragen. Ein Herabtropfen von Flüssigkeit aus dem Testträger oder ein Kontakt von Flüssigkeit beispielsweise mit Teilen eines Gerätes, in das der Testträger zur apparativen Auswertung gelegt wird, wird zuverlässig vermieden. Dies ist insbesondere bei der Untersuchung von Blut oder von Blut abgeleiteten Proben, wie Plasma oder Serum, ein sehr wichtiger Aspekt.

Die Größe des über die Nachweisschicht hinausragenden Bereiches des Netzwerkes richtet sich nach dem in der Praxis größten zu erwartenden Probenvolumen, damit auch tatsächlich überschüssige Flüssigkeit von der Nachweisschicht weggeführt werden kann. Auf diese Art und Weise ist die bei Anwesenheit eines Analyts sich einstellende Signalintensität unabhängig von der Menge und der Dauer des Kontaktes der Probenflüssigkeit mit der Nachweisschicht. Die Farbe, die sich nach Beendung der Nachweisreaktion, üblicherweise innerhalb weniger Sekunden bis weniger Minuten eingestellt hat, bleibt so für die Messung unverändert. Sie wird lediglich durch die Stabilität des farbgebenden Systems bestimmt, nicht aber beispielsweise durch Analyt, der aus überschüssiger Flüssigkeit in die Nachweisschicht nachdifundiert. Falsch positive Resultate werden so ebenfalls vermieden und eine quantitative Analytbestimmung ermöglicht.

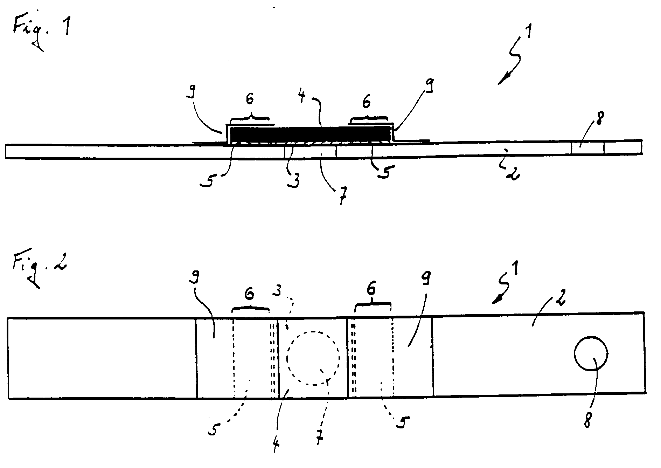

Eine bevorzugte Ausführungsform des erfindungsgemäßen diagnostischen Testträgers ist in Fig. 1 und 2 dargestellt.

Fig. 1 stellt einen Querschnitt durch einen bevorzugten erfindungsgemäßen diagnostischen Testträger dar.

Fig. 2 zeigt die Aufsicht auf den in Fig. 1 im Querschnitt dargestellten erfindungsgemäßen diagnostischen Testträger.

Die in den Figuren verwendeten Bezugszeichen bedeuten:
1) Diagnostischer Testträger
2) Tragschicht
3) Nachweisschicht
4) Netzwerk
5) Befestigungsschicht
6) Über die Nachweisschicht hinausragender Bereich des Netzwerkes
7) Loch unter Nachweisschicht in der Tragschicht
8) Positionierloch.
9) Abdeckung

Der in Fig. 1 dargestellte Querschnitt durch und die in Fig. 2 gezeigte Aufsicht auf einen erfindungsgemäßen diagnostischen Testträger (1) zeigt eine Tragschicht (2) mit der Nachweisschicht (3) und dem die Nachweisschicht (3) überdeckenden Netzwerk (4), welches im über die Nachweisschicht (3) hinausragenden Bereich (6) auf der Tragschicht (2) mit einer Befestigungsschicht (5) befestigt ist. Unter der Nachweisschicht (3) befindet sich ein Loch (7) in der Tragschicht (2), durch das die Nachweisschicht (3) sichtbar ist. Das Positionierloch (8) dient dazu, den Teststreifen im Falle einer apparativen, beispielsweise einer reflektionsphotometrischen Vermessung an einer genau vorbestimmten Stelle des Apparates festzuhalten. Dies kann dadurch geschehen, daß beispielsweise ein Stift in das Positionierloch (8) hineinragt und so den Teststreifen (1) an einer vorbestimmten Stelle festhält. Die Abdeckfolie (9) ist auf der Tragschicht (2) befestigt und überdeckt den über die Nachweisschicht (3) hinausragenden Bereich (6) des Netzwerkes (4), so daß der Bereich des Netzwerkes (4), der über der Nachweisschicht (3) liegt, frei bleibt.

### Beispiel 1

### Herstellung eines erfindungsgemäßen diagnostischen Testträgers

Die Herstellung eines Testträgers gemäß Fig. 1 erfolgt mit folgenden Arbeitsschritten:

Auf eine Titandioxid-haltige Polyester-Tragschicht wird ein 6 mm breites doppelseitiges Klebeband (Polyesterträger und Synthesekautschuk-Kleber) aufgebracht. Dieser Verbund wird mit einem Lochabstand von 6 mm gemeinsam gestanzt, um die Meßlöcher zu erzeugen. Danach wird das Schutzpapier des doppelseitigen Klebebands abgezogen.

Zur Herstellung eines Nachweisfeldes, das aus 2 Filmschichten aufgebaut ist, wird so vorgegangen:
A. In einem Becherglas werden die folgenden Komponenten als Reinsubstanzen oder in Form von Stammlösungen in folgender Zusammensetzung zusammengegeben und durch Rühren gemischt:

| | |
|---|---|
| Wasser: | 820,0 g |
| Citronensäure- 1-hydrat: | 2,5 g |
| Calciumchlorid-2-hydrat: | 0,5 g |
| Natriumhydroxid: | 1,4 g |
| Xanthan gum: | 3,4 g |
| Tetraethylammoniumchlorid: | 2,0 g |
| N-Octanoyl-N-methyl-glucamid: | 2,1 g |
| Polyvinylpyrrolidon (MG 25000): | 3,5 g |
| Transpafill® (Natrium-Aluminiumsilikat): | 62,1 g |
| Polyvinylpropionat-Dispersion (50 Gew.-% in Wasser): | 60,8 g |
| Bis-(2-hydroxyethyl)-(4-hydroximinocyclohexa-2,5-dienylidin)ammoniumchlorid: | 1,2 g |
| 2,18-Phosphormolybdänsäure-hexanatriumsalz: | 16,1 g |
| Pyrrolochinolin-chinon: | 32 mg |
| Glucosedehydrogenase rec. aus Acinetobacter calcoaceticus, EC 1.1.99.17: | 1,7 MU (2,4 g) |
| 1-Hexanol: | 1,6 g |
| 1-Methoxy-2-propanol: | 20,4 g |

Die Gesamtmasse wird mit NaOH auf einen pH von ca. 6 eingestellt und dann mit einem Flächengewicht von 89 g/qm auf eine 125 µm dicke Polycarbonatfolie aufgetragen und getrocknet.
B. In einem Becherglas werden die folgenden Komponenten als Reinsubstanzen oder in Form von Stammlösungen in folgender Zusammensetzung zusammengegeben und durch Rühren gemischt:

| | |
|---|---|
| Wasser: | 579,7 g |
| Natriumhydroxid: | 3,4 g |
| Gantrez® (Methylvinylether-maleinsäure-Copolymer): | 13,8 g |
| N-Octanoyl-N-methyl-glucamid: | 3,6 g |
| Tetraethylammoniumchlorid: | 9,7 g |
| Polyvinylpyrrolidon (MG 25000): | 20,2 g |
| Titandioxid: | 177,1 g |
| Kieselgur: | 55,3 g |
| Polyvinylpropionat-Dispersion (50 Gew.-% in Wasser: | 70,6 g |
| 2,18-Phosphormolybdänsäure-hexanatriumsalz: | 44,3 g |
| Kaliumhexacyanoferrat (III): | 0,3 g |
| 1-Hexanol: | 1,6 g |
| 1-Methoxy-2-propanol: | 20,4 g |

Die Gesamtmasse wird mit NaOH auf einen pH von ca. 6 eingestellt und dann mit einem Flächengewicht von 104 g/qm auf die wie vorstehend unter A. beschriebene beschichtete Polycarbonatfolie aufgetragen und getrocknet.

Ein 5 mm breiter Streifen der so hergestellten Nachweisschicht wird mit der Folienseite auf das gestanzte doppelseitige Klebeband auf der Tragschicht passgenau aufgeklebt.

Direkt an die Nachweisschicht angrenzend werden aufbeiden Seiten doppelseitige Klebebänder (PVC-Träger und Naturkautschuk-Kleber) als Abstandshalter und Befestigungsschicht auf die Trägerfolie aufgeklebt. Im vorliegenden Beispiel ist ein Abstandshalter 6 mm und der andere 9 mm breit. Danach wird die Schutzfolie der beiden doppelseitigen Klebebänder abgezogen.

Auf diesen Verbund wird ein mit Netzmittel imprägniertes Gewirke Doubleface 450 (Firma Karl Otto Braun, Wolfstein, Deutschland) gemäß DE-C-32 13 673 als Netzwerk aufgelegt und durch Anpressen verklebt.

Es werden zwei einseitige Klebebänder (PVC-Träger und Naturkautschuk-Kleber) als Abdeckungen so auf das Netzwerk aufgeklebt, daß die Abstandshalter ganz abgedeckt werden und wenigstens noch eine geringfügige Überlappung mit dem Reaktivbezirk stattfindet. Damit ist die Bandware fertiggestellt.

Die Bandware wird so in 6 mm breite Testträger geschnitten, daß das Meßloch im Testträger mittig liegt.

Mit unterschiedlichen Blutvolumina (5,8, 10 und 15 µl) werden mit solchermaßen hergestellten Testträgern in der Nachweisschicht stets gleichmäßige und reproduzierbare zeitlich stabile Farben erzeugt, die der Glucosemenge im Blut proportional sind. Die Volumenabhängigkeit ist kleiner 2% bezogen aufeinen Remissionsmeßwert, der mit 10 µl Probe erhalten wird.

## Patentansprüche

1. Diagnostischer Testträger (1) enthaltend eine Tragschicht (2) mit einer darauf angeordneten zur Bestimmung eines Analyts in flüssiger Probe erforderliche Reagenzien enthaltenden Nachweisschicht (3) und ein die Nachweisschicht (3) überdeckendes Netzwerk (4), das größer als die Nachweisschicht (3) ist und das auf der Tragschicht (2) befestigt ist,
dadurch gekennzeichnet, daß das Netzwerk (4) ein mehrfädiges Gewirke ist, dessen Fäden an der der Nachweisschicht (3) zugewandten Seite stark aufgerauht sind, während die Fäden an der der Nachweisschicht (3) abgewandten Seite des Gewirkes relativ glatt sind.

2. Diagnostischer Testträger (1) gemäß Patentanspruch 1, dadurch gekennzeichnet, daß das Netzwerk (4) ein elastisches Gewirke mit einem maschenbildenden Faden aus einem ersten Material und mit als Schußfäden in das Flächengebilde eingearbeiteten Fäden aus einem sehr saugfähigen zweiten Material ist, wobei das zweite Material hydrophiler als das erste Material ist und
sich der maschenbildende Faden auf der der Nachweisschicht (3) abgewandten Seite des Gewirkes befindet und die Schußfäden auf der der Nachweisschicht (3) zugewandten Seite des Gewirkes.

3. Diagnostischer Testträger (1) gemäß Patentanspruch 2, dadurch gekennzeichnet, daß die Schußfäden aus sehr saugfähigen Garnen bestehen, die auf der der Nachweisschicht (3) zugewandten Seite des Gewirkes stark aufgerauht sind.

4. Diagnostischer Testträger (1) gemäß Patentanspruch 2, dadurch gekennzeichnet, daß der maschenbildende Faden aus synthetischem Material besteht.

5. Diagnostischer Testträger (1) gemäß einem der Patentansprüche 1-4, dadurch gekennzeichnet, daß Nachweisschicht (3) und diese überdeckendes Netzwerk (4) so angeordnet sind, daß von der Nachweisschicht (3) ein kapillaraktiver Flüssigkeitstransport in den Teil des Netzwerkes möglich ist, der auf der Tragschicht (2) befestigt ist, insbesondere wenn die Nachweisschicht (3) mit Flüssigkeit gesättigt ist.

6. Diagnostischer Testträger (1) gemäß einem der Patentansprüche 1-5, dadurch gekennzeichnet, daß sich über dem die Nachweisschicht (3) überragenden Bereich (6) des Netzwerkes (4) eine Abdeckung (9) befindet, die so angeordnet ist, daß der Bereich des Netzwerkes (4), der über der Nachweisschicht (3) liegt, frei bleibt.

7. Diagnostischer Testträger (1) gemäß einem der Patentansprüche 1-6, dadurch gekennzeichnet, daß die Nachweisschicht (3) durch die Tragschicht (2) sichtbar ist.

8. Diagnostischer Testträger (1) gemäß Patentanspruch 7, dadurch gekennzeichnet, daß die Tragschicht (2) ein Loch (7) aufweist, das von der Nachweisschicht (3) überdeckt ist.

9. Verwendung eines diagnostischen Testträgers (1) gemäß einem der Patentansprüche 1-8 zur Bestimmung eines Analyten in einer Flüssigkeit.

10. Verfahren zur Bestimmung eines Analyts in flüssiger Probe mit Hilfe eines diagnostischen Testträgers (1) gemäß einem der Patentansprüche 1-8, wobei so viel Probenflüssigkeit auf die der Nachweisschicht (3) abgewandte Seite des Netzwerkes (4) aufgegeben wird, daß die Nachweisschicht (3) mit Flüssigkeit gesättigt wird und das Netzwerk (4) überschüssige Flüssigkeit von der Nachweisschicht (3) in den die Nachweisschicht (4) überragenden Bereich des Netzwerkes (6) wegführt und die Nachweisschicht (3) auf eine Farbveränderung hin beobachtet wird, wobei die Intensität der Farbveränderung ein Maß für die Menge an Analyt in der untersuchten flüssigen Probe darstellt.

11. Verwendung eines Flüssigkeit aufnehmenden flächig ausgebildeten Gewirkes mit einem maschenbildenden Faden aus einem vollsynthetischen Material und aus als Schußfäden in das Gewirke eingearbeiteten hydrophilen Fäden aus saugfähigen Garnen, die auf einer Seite des Gewirkes aufgerauht sind
als Flüssigkeit verteilende Schicht in einem diagnostischen Testträger (1).
